# EUROPÄISCHE PATENTANMELDUNG

(11) **EP 1 894 954 A1**
(43) Veröffentlichungstag der Anmeldung: **05.03.2008**
(21) Anmeldenummer: 07114619.5
(22) Anmeldetag: 20.08.2007
(51) Int. Cl.: C08G 18/12, C08G 18/76, C07C 263/20

(54) **Isocyanatkomponente daraus hergestellte Prepolymere sowie Polyurethan Ein- und Zweikomponentenschäume daraus**

(30) Priorität: 01.09.2006 EP 06119986
(71) Anmelder: BASF SE, 67056 Ludwigshafen (DE)
(72) Erfinder: Reese, Hans-Jürgen, 49401, Damme (DE); Murrar, Imbridt, 01968, Senftenberg (DE); Fritz, Ralf, 49143, Bissendorf-Schledehausen (DE); Cabrera, Andres, 49080, Osnabrück (DE); Magg, Birgit, 49401, Damme (DE)

(57) **Zusammenfassung**

Gegenstand der Erfindung ist eine Isocyanatkomponente (B), enthaltend höhere Homologe des Diphenylmethandiisocyanats, enthaltend
(B1) das 3-Kern-Produkt des Polyphenylenpolymethylenpolyisocyanats,
(B2) das 4-Kern-Produkt des Polyphenylenpolymethylenpolyisocyanats,
(B3) das 5-Kern-Produkt des Polyphenylenpolymethylenpolyisocyanats

wobei diese in einem Gewichtsverhältnis der Komponenten (B1) : (B2) : (B3) = 8,0±4,0 : 3,5±1,8 : 1,2±0,9 vorliegen, und die Isocyanatkomponente (B) einen Gehalt an Diphenylmethandiisocyanat von maximal 2 Gew.-% und einen Gehalt an Uretoniminen von maximal 6 Gew.-%, jeweils bezogen auf das Gewicht der Isocyanatkomponente (B), aufweist, daraus hergestellte Prepolymere sowie Polyurethan Ein- und Zweikomponentenschäume daraus.

## Beschreibung

Gegenstand der Erfindung sind Isocyanatgruppen und Urethangruppen enthaltende Prepolymere, ihre Herstellung sowie ihre Verwendung zur Herstellung von Polyurethanen, insbesondere 1-Komponenten-Polyurethan-Montageschäumen.

1-Komponenten-Polyurethan-Montageschäume, im folgenden auch als Einkomponentenschäume bezeichnet, aus Aerosolbehältern sind im Bereich des Bauwesens häufig angewandte Montagemittel zum Einbau von Fenstern und Türen in Bauwerken sowie als Füllmaterial für bautechnisch bedingte Hohlräume oder Mauerdurchbrüche für Rohrinstallationen. Ein solcher Aerosolbehälter beinhaltet ein Isocyanatgruppen und Urethangruppen enthaltendes Prepolymer sowie Treibmittel und Zusätze. Durch Austragen des Inhalts der Aerosoldose mittels Treibmittel, dessen Aufschäumen durch Frothwirkung und durch seine Aushärtung mit Luftfeuchtigkeit, entsteht der gewünschte Schaum.

Einkomponentenschäume auf der Basis von NCO-haltigen Prepolymeren sind die bekanntesten Schäume dieser Art. Es gibt hierbei unterschiedliche Produkte, die je nach Zusammensetzung zu harten bis weich-elastischen Schäumen führen.

Nachteilig bei vielen dieser Formulierungen ist, dass erhebliche Mengen an monomerem Isocyanat in diesen NCO-haltigen Prepolymeren vorhanden ist und damit während des Schäumprozesses ein gewisses Gefährdungspotential durch atembares Isocyanat besteht. Bekannt sind in dieser Gruppe von Schäumen aber auch Formulierungen mit deutlich reduzierten Gehalten an freien monomeren Isocyanaten.

So ist aus EP 1 518 874 ein monomerarmes Isocyanat zur Fertigung von Einkomponentenschäumen bekannt, welches aus einem definierten Polyphenylen-polymethylenpolyisocyanat durch destillative Entfernung des monomeren Isocyanats gewonnen wird. Durch Einsatz dieses Produktes, gegebenenfalls im Gemisch mit Verdünnungsmitteln und weiteren isocyanatgruppenhaltigen Verbindungen werden so monomerarme Einkomponentenschäume erhalten. Nachteilig hierbei ist, dass so gefertigte Einkomponenten-Aerosolschäume wenig lagerstabil sind, wodurch der Inhalt der Aerosoldruckbehälter innerhalb weniger Wochen fest und damit unbrauchbar wird.

Auch die WO 2005/007721 A1 beschreibt den Einsatz von Gemischen aus monomerarmen NCO-endständigen Prepolymeren, d. h. von Monomeren befreiten Umsetzungsprodukten aus Polyolen und Diphenylmethandiisocyanat im stöchiometrischen Überschuß, entmonomerisierten Polyphenylen-polymethylenpolyisocyanat, trimerisierten Hexamethylendiisocyanat und Verdünnungsmitteln. Nachteilig sind hier die zur Realisierung des geforderten verminderten Monomergehaltes an Isocyanat extrem hohen Viskositäten der Einsatzstoffe, die den Einsatz technologisch schwierig gestalten und die Tatsache, dass die Lagerstabilität wie bei der Lösung nach EP 1 518 874 nicht gewährleistet ist.

Auch bei Polyurethan-Hartschaumstoffen werden vom Markt zunehmend Produkte verlangt, die eine hellere Farbe aufweisen. Außerdem besteht die ständige Forderung, die Gebrauchseigenschaften sowie die Verarbeitungseigenschaften der Produkte zu verbessern. Insbesondere die Dimensionsstabilität der Schäume hoch sein, das heißt der Schrumpf sollte möglichst gering sein.

Es war die Aufgabe der vorliegenden Erfindung, Polyurethane, insbesondere Polyurethan-Schaumstoffe bereitzustellen, die eine geringe Farbe sowie gute Gebrauchs- und Verarbeitungseigenschaften aufweisen. Insbesondere sollten Einkomponentenschäume, die einen geringen Anteil an monomerem Diisocyanat aufweisen, eine gute Lagerstabilität zeigen und zu Schaumstoffen mit guten Gebrauchseigenschaften umgesetzt werden können, sowie helle Polyurethan-Hartschaumstoffe bereitgestellt werden.

Es wurde überraschenderweise gefunden, dass bei Verwendung einer Isocyanatkomponente aus höheren Homologen des Diphenylmethandiisocyanats (MDI) mit einer definierten Zusammensetzung der 3-, 4- und 5-Kern-Homologen des Diphenylmethandiisocyanats, einem geringen Gehalt an 2-Kern-Diphenylmethandiisocyanat und einem geringen Gehalt an Uretonimin als Isocyanatkomponente zur Herstellung der Hartschäume sowie des Prepolymeren für den Einkomponentenschaum die Aufgabe der Erfindung gelöst werden konnte. Außerdem hat sich gezeigt, dass bei der Verwendung dieser Isocyanatkomponente Schäume resultieren, die sehr hell, häufig sogar weiß sind.

Gegenstand der Erfindung sind daher eine Isocyanatkomponente (B), enthaltend höhere Homologe des Diphenylmethandiisocyanats, enthaltend
(B1) das 3-Kern-Produkt des Polyphenylenpolymethylenpolyisocyanats,
(B2) das 4-Kern-Produkt des Polyphenylenpolymethylenpolyisocyanats,
(B3) das 5-Kern-Produkt des Polyphenylenpolymethylenpolyisocyanats
wobei diese in einem Gewichtsverhältnis der Komponenten (B1) : (B2) : (B3) = 8,0±4,0 : 3,5±1,8 : 1,2±0,9 vorliegen, und die Isocyanatkomponente (B) einen Gehalt an Diphenylmethandiisocyanat von maximal 2 Gew.-% und einen Gehalt an Uretoniminen von maximal 6 Gew.-%, jeweils bezogen auf das Gewicht der Isocyanatkomponente (B), aufweist.

Gegenstand der Erfindung sind weiterhin Isocyanatgruppen und Urethangruppen enthaltende Prepolymere, hergestellt durch Umsetzung einer Polyolkomponente (A) und einem stöchiometrischen Überschuss einer Isocyanatkomponente (B), dadurch gekennzeichnet, dass
die Isocyanatkomponente (B) höhere Homologe des Diphenylmethandiisocyanats, enthaltend
(B1) das 3-Kern-Produkt des Polyphenylenpolymethylenpolyisocyanats,
(B2) das 4-Kern-Produkt des Polyphenylenpolymethylenpolyisocyanats,
(B3) das 5-Kern-Produkt des Polyphenylenpolymethylenpolyisocyanats
wobei diese in einem Gewichtsverhältnis der Komponenten (B1) : (B2) : (B3) = 8,0±4,0 : 3,5±1,8 : 1,2±0,9 vorliegen, und die Isocyanatkomponente (B) einen Gehalt an Diphenylmethandiisocyanat von maximal 2 Gew.-% und einen Gehalt an Uretoniminen von maximal 6 Gew.-%, jeweils bezogen auf das Gewicht der Isocyanatkomponente (B), aufweist
enthält.

Gegenstand der Erfindung ist weiterhin die Verwendung der erfindungsgemäßen Isocyanatgruppen und Urethangruppen enthaltendes Prepolymere zur Herstellung von hellen Polyurethanschaumstoffen, insbesondere von 1-Komponenten-Montageschaumstoffen.

Gegenstand der Erfindung ist weiterhin ein Verfahren zur Herstellung von 1-Komponenten-Montageschaumstoffen durch Umsetzung einer Polyolkomponente (A) und einem stöchiometrischen Überschuss einer Isocyanatkomponente (B), dadurch gekennzeichnet,
die Isocyanatkomponente (B) höhere Homologe des Diphenylmethandiisocyanats, enthaltend
(B1) das 3-Kern-Produkt des Polyphenylenpolymethylenpolyisocyanats,
(B2) das 4-Kern-Produkt des Polyphenylenpolymethylenpolyisocyanats,
(B3) das 5-Kern-Produkt des Polyphenylenpolymethylenpolyisocyanats
wobei diese in einem Gewichtsverhältnis der Komponenten (B1) : (B2) : (B3) = 8,0±4,0 : 3,5±1,8 : 1,2±0,9 vorliegen, dabei die Isocyanatkomponente (B) einen Gehalt an Diphenylmethandiisocyanat von maximal 2 Gew.-% und einen Gehalt an Uretoniminen von maximal 6 Gew.-%, jeweils bezogen auf das Gewicht der Isocyanatkomponente (B), aufweist und die Komponenten (A) und (B) in Gegenwart von Flüssiggasen in einem Druckbehälter umgesetzt werden.

Gegenstand der Erfindung ist weiterhin ein Verfahren zur Herstellung von Polyurethan-Hartschaumstoffen durch Umsetzung von Verbindungen mit mindestens zwei mit Isocyanatgruppen reaktiven Wasserstoffatomen mit einer Isocyanatkomponente (B), dadurch gekennzeichnet, dass die Isocyanatkomponente (B) höhere Homologe des Diphenylmethandiisocyanats enthält, enthaltend
(B1) das 3-Kern-Produkt des Polyphenylenpolymethylenpolyisocyanats,
(B2) das 4-Kern-Produkt des Polyphenylenpolymethylenpolyisocyanats,
(B3) das 5-Kern-Produkt des Polyphenylenpolymethylenpolyisocyanats
   wobei diese in einem Gewichtsverhältnis der Komponenten (B1) : (B2) : (B3) = 8,0±4,0 : 3,5±1,8 : 1,2±0,9 vorliegen, und die Isocyanatkomponente (B) einen Gehalt an Diphenylmethandiisocyanat von maximal 2 Gew.-% und einen Gehalt an Uretoniminen von maximal 6 Gew.-%, jeweils bezogen auf das Gewicht der Isocyanatkomponente (B), aufweist.

Unter dem Begriff "Kern" wird hierbei ein aromatischer Ring verstanden.

Der Gehalt von Uretoniminen in Polymer-MDI wird mittels FT-IR-Analyse auf der Grundlage einer Kalibration mit 3-Kern Uretonimin ermittelt (Prüfverfahren PFO/A 00/22-03).

Die für das erfindungsgemäße Verfahren verwendete Isocyanatkomponente (B) weist dabei vorzugsweise eine Jodfarbzahl von kleiner 5 Jod, einen L*-Wert von größer 96 und einen b*-Wert von kleiner 15 auf, bestimmt nach DIN 6162 und DIN 6164.

Der NCO-Gehalt der Prepolymere liegt vorzugsweise im Bereich von ca. 5 bis 28 Gew.-%, vorzugsweise 8 bis 24 Gew.-%, besonders bevorzugt 9 bis 18 Gew.-%. Prepolymere mit niederen NCO-Gehalt führen zu weicheren Aerosolschäumen. Entsprechend solche mit höherem NCO-Gehalt zu härteren Schäumen.

In einer bevorzugten Ausführungsform des erfindungsgemäßen Verfahren besteht die Isocyanatkomponente (B) zu mehr als 85 Gew.-%, bezogen auf das Gewicht der Isocyanatkomponente (B), aus den Komponenten (B1), (B2) und (B3). Der Rest der Isocyanatkomponente (B), der unter Einbeziehung der Gehalte an 2-Kern-Diphenylmethandiisocyanat und Uretonimin zu 100 Gew.-% fehlt, sind zumeist Homologe des Diphenylmethandüsocyanats mit 6 oder mehr Kernen. Unter höheren Homologen des Diphenylmethandüsocyanats werden hierbei Polyphenylenpolymethylenpolyisocyanate mit drei oder mehr aromatischen Kernen verstanden.

Als Verbindungen mit mindestens zwei gegenüber Isocyanat reaktiven Wasserstoffatomen (A), die für das erfindungsgemäße Verfahren verwendet werden können, kommen bei der Herstellung der Hartschaumstoffe und bei der Herstellung der Prepolymere für die 1-Komponenten-Montageschaumstoffe insbesondere Polyetheralkohole und/oder Polyesteralkohole mit OH-Zahlen im Bereich von 100 bis 1200 mgKOH/g zum Einsatz.

Die eingesetzten Polyesteralkohole werden zumeist durch Kondensation von mehrfunktionellen Alkoholen, vorzugsweise Diolen, mit 2 bis 12 Kohlenstoffatomen, vorzugsweise 2 bis 6 Kohlenstoffatomen, mit mehrfunktionellen Carbonsäuren mit 2 bis 12 Kohlenstoffatomen, beispielsweise Bernsteinsäure, Glutarsäure, Adipinsäure, Korksäure, Azelainsäure, Sebacinsäure, Decandicarbonsäure, Maleinsäure, Fumarsäure und vorzugsweise Phthalsäure, Isophthalsäure, Terephthalsäure und die isomeren Naphthalindicarbonsäuren, hergestellt.

Die verwendeten Polyetheralkohole haben zumeist eine Funktionalität zwischen 2 und 8, insbesondere 3 bis 8.

Insbesondere kommen Polyetherpolyole, die nach bekannten Verfahren, beispielsweise durch anionische Polymerisation von Alkylenoxiden in Gegenwart von Katalysatoren, vorzugsweise Alkalihydroxiden, hergestellt werden, zum Einsatz.

Als Alkylenoxide werden zumeist Ethylenoxid und/oder Propylenoxid, vorzugsweise reines 1,2-Propylenoxid eingesetzt.

Als Startmoleküle kommen insbesondere Verbindungen mit mindestens 3, vorzugsweise 4 bis 8 Hydroxylgruppen oder mit mindestens zwei primären Aminogruppen im Molekül zum Einsatz.

Als Startmoleküle mit mindestens 3, vorzugsweise 4 bis 8 Hydroxylgruppen im Molekül werden vorzugsweise Trimethylopropan, Glycerin, Pentaerythrit, Zuckerverbindungen wie beispielsweise Glucose, Sorbit, Mannit und Saccharose, mehrwertige Phenole, Resole, wie z. B. oligomere Kondensationsprodukte aus Phenol und Formaldehyd und Mannich-Kondensate aus Phenolen, Formaldehyd und Dialkanolaminen sowie Melamin eingesetzt.

Als Startmoleküle mit mindestens zwei primären Aminogruppen im Molekül werden vorzugsweise aromatische Di- und/oder Polyamine, beispielsweise Phenylendiamine, 2,3-, 2,4-, 3,4- und 2,6-Toluylendiamin und 4,4'-, 2,4'- und 2,2'-Diamino-diphenylmethan sowie aliphatische Di- und Polyamine, wie Ethylendiamin, eingesetzt.

Die Polyetherpolyole besitzen eine Funktionalität von vorzugsweise 3 bis 8 und Hydroxylzahlen von vorzugsweise 100 mgKOH/g bis 1200 mgKOH/g und insbesondere 240 mgKOH/g bis 570 mgKOH/g.

Es können zusätzlich auch Polyole, insbesondere Polyetheralkohole mit einer Hydroxylzahl von kleiner 100 mgKOH/g und einer Funktionalität von 2 bis 3 eingesetzt werden. dadurch können die Eigenschaften der Schaumstoffe eingestellt werden, beispielsweise bei 1-Komponenten-Montageschäumen in Richtung einer höheren Flexibilität.

Zu den Verbindungen mit mindestens zwei gegenüber Isocyanat reaktiven Wasserstoffatomen b1) gehören auch die gegebenenfalls mitverwendeten Kettenverlängerer und Vernetzer. Zur Modifizierung der mechanischen Eigenschaften kann sich der Zusatz von difunktionellen Kettenverlängerungsmitteln, tri- und höherfunktionellen Vernetzungsmitteln oder gegebenenfalls auch Gemischen davon als vorteilhaft erweisen. Als Kettenverlängerungs- und/oder Vernetzungsmittel verwendet werden vorzugsweise Alkanolamine und insbesondere Diole und/oder Triole mit Molekulargewichten kleiner als 400, vorzugsweise 60 bis 300.

Kettenverlängerungsmittel, Vernetzungsmittel oder Mischungen davon werden zweckmäßigerweise in einer Menge von 1 bis 20 Gew.-%, vorzugsweise 2 bis 5 Gew.-%, bezogen auf die Polyolkomponente b), eingesetzt.

Weitere Angaben zu den verwendeten Polyetheralkoholen und Polyesteralkoholen sowie ihrer Herstellung finden sich beispielsweise im Kunststoffhandbuch, Band 7 "Polyurethane", herausgegeben von Günter Oertel, Carl-Hanser-Verlag München, 3. Auflage, 1993.

In einer besonders bevorzugten Ausführungsform der Herstellung der erfindungsgemäßen Prepolymere enthält die zur Herstellung der Prepolymere eingesetzte Polyolkomponente (A) ein Gemisch aus
(A1) einem Polyesterpolyol mit einem Molekulargewicht von maximal 600 g/mol und
(A2) einem Polyetherpolyol oder Polyetherpolyolgemisch mit einem mittleren Molekulargewicht von 1000 bis 5000 g/mol.

Als Polyesterpolyol (A1) wird dabei vorzugsweise ein Polyesterpolyol auf Basis von Phthalsäureanhydrid / Diethylenglykol / Polyethylenglykol verwendet.

Die Polyole (A1) und (A2) werden bevorzugt in einem Gewichtsverhältnis von Polyesterpolyol (A1)zu Polyetherpolyol oder Polyetherpolyolgemisch (A2) im Bereich von 1 : 6 bis 3 : 1 eingesetzt.

Zur Herstellung der erfindungsgemäß eingesetzten Isocyanatkomponente (B) wird das Roh-MDI von monomerem Diisocyanat und von Uretonimin befreit. Die erfindungsgemäße Isocyanatkomponente (B) wird daher üblicherweise hergestellt, indem aus handelsüblichen Gemischen aus Diphenylmethandiisocyanat und Polyphenylenpolymethylenpolyisocyanaten, die häufig auch als Roh-MDI oder Polymer-MDI bezeichnet werden und die beispielsweise als Lupranat® M-Typen von der BASF AG, beispielsweise unter der Bezeichnung Lupranat® M20 oder Lupranat® M50, vertrieben werden, das monomere Diisocyanat und die Uretonimine abgetrennt werden.

Wie beschrieben, wird das zur Herstellung der Isocyanatkomponente (B) eingesetzte Gemisch aus Diphenylmethandiisocyanat und Polyphenylenpolymethylenpolyisocyanaten zunächst von Nebenverbindungen, wie Uretonimin befreit. Diese werden bei der Herstellung und Aufarbeitung insbesondere durch thermische Belastung der Polyisocyanate gebildet. Diese Nebenverbindungen aus dem Herstellungsprozess, wie Uretdione, Uretonimine, Carbamoylchloride, sind in dem Ausgangspolyisocyanat zu maximal 25 Gew.-% enthalten. Die Entfernung erfolgt vorzugsweise durch Flüssig-Flüssig-Extraktion mit polaren oder unpolaren Lösungsmitteln. In einer besonderen Ausführungsform bevorzugt sind Kohlenwasserstoffe, wie Cyclohexan. Derartige Verfahren sind beispielsweise beschrieben in DE 15 43 258 oder EP 133 538.

In einer bevorzugten Ausführungsform der Abtrennung des Uretonimins wird das eingesetzte Polyphenylenpolymethylenpolyisocyanat mit Cyclohexan im Verhältnis von Isocyanat: Lösungsmittel von 1 : 1 bis 1 : 15, bevorzugt 1 : 1,5 bis 1 : 12 und besonders bevorzugt 1 : 2,5 bis 1 : 10 bei einer Temperatur von 20 bis 90 °C und bevorzugt 30 bis 80 °C für 1 bis 180 min und bevorzugt 5 bis 150 min in Kontakt gebracht. Danach wird das Produktgemisch bis zur vollständigen Phasenbildung bei 20 bis 40 °C und bevorzugt bei Raumtemperatur stehen gelassen. Die untere Phase ist das sogenannte "Raffinat", das das abzutrennende Uretonimin sowie höherkernige MDI-Homologe enthält. Die obere Phase ist das sogenannte "Extrakt", das das gewünschte uretoniminarme Polyphenylenpolymethylenpolyisocyanat und Lösungsmittel enthält. Beide Phasen werden getrennt und das Lösungsmittel beispielsweise mittels Vakuumdestillation praktisch vollständig entfernt. Der Restgehalt an Cyclohexan ist vorzugsweise kleiner 20 ppm.

Aus dem so behandelten Gemisch wird anschließend das monomere Diisocyanat abgetrennt. Die Entfernung des monomeren Diisocyanats kann vorzugsweise durch Destillation, vorzugsweise unter Vakuum, erfolgen. Dabei ist es bevorzugt, die Destillation mit einem Dünnschichtverdampfer oder einem Kurzwegverdampfer durchzuführen. Ein derartiges Verfahren ist beispielsweise in EP 1 518 874 beschrieben. Die Abtrennung der Monomere wird vorzugsweise bei einer Temperatur von unter 160 °C, besonders bevorzugt 100 bis 158 °C und insbesondere 120 bis 155 °C durchgeführt. Der Druck beträgt vorzugsweise 0,001 bis 10 mbar, besonders bevorzugt 0,01 bis 1 mbar und insbesondere 0,02 bis 0,9 mbar.

Es kann zwar prinzipiell auch in umgekehrter Reihenfolge verfahren werden, hierbei können jedoch verstärkt Nebenreaktionen ablaufen, die zu Produktverlusten führen können. Außerdem kann ein Teil der gebildeten Nebenprodukte nur noch schwierig aus dem Polyisocyanat entfernt werden.

Durch die Einstellung der Bedingungen bei der Extraktion und der Destillation kann das gewünschte Verhältnis der Komponenten (B1), (B2) und (B3) zueinander eingestellt werden.

### Das erfindungsgemäße Prepolymer weist eine niedrige Viskosität auf

Bei Verwendung der besonders bevorzugten Polyolkomponente (A), enthaltend die Komponenten (A1) und (A2), kann die Viskosität trotz paralleler Anwesenheit von Polyesterpolyolen und Polyetherpolyolen weiter gesenkt werden, sowie der Gehalt an verträglichkeits-verbessernden Verdünnungsmitteln stark reduziert bis verzichtet werden.

Es ist jedoch für viele Anwendungen erforderlich, additive Flammschutzmittel zuzugeben, die ebenfalls viskositätssenkend wirken. Dieses muss nur noch in einer solchen Menge zugegeben werden, wie es zur Erreichung der Brandtklasse des fertigen Schaums notwendig ist

Als additive Flammschutzmittel werden zumeist Trialkylphosphate sowie Trichloralkylphosphate eingesetzt. Die Alkylreste haben vorzugsweise 1 bis 4, besonders bevorzugt 1 bis 3 Kohlenstoffatome. Besonders bevorzugte Verbindungen sind Trimethylphosphat, Triethylphosphat, Tripropylphosphat, Trichlormethylphosphat, Trichlorethylphospat und Trichlorpropylphosphat. Diese können einzeln oder in beliebigen Gemischen untereinander eingesetzt werden

Die Menge der additiven Flammschutzmittel hängt von den Anforderungen an den Schaum ab.

Für die Formulierung von flammgeschützten Aerosolschäumen muss das in der Dose befindliche NCO-Prepolymer in der Summe einen Gehalt an additiven Flammschutzmitteln von ca. 8 bis 18 Gew.-%, vorzugsweise 12 bis 16 Gew.-%, bezogen auf das Gewicht des Prepolymeren, haben. Bei geringeren Gehalten kann der Flammschutz nicht ausreichend sein, bei zu hohen Gehalten an additiven Flammschutzmittel verfließt der Schaum, das heißt er ist nicht senkrecht applizierbar, oder das Flammschutzmittel migriert in zu großen Maße aus dem Schaum.

Überraschenderweise hat sich gezeigt, dass die Komponenten (A1) und (A2) in einem weiten Bereich gut miteinander mischbar sind. Dies ist insofern besonders überraschend, als Polyetheralkohole und Polyesteralkohole normalerweise schlecht miteinander mischbar sind. In den üblichen Verfahren kann die Mischbarkeit durch Zusatz der additiven Flammschutzmittel verbessert werden. Das erfindungsgemäße Polyolgemisch zeigt auch nach Zusatz von sehr geringen Mengen an Flammschutzmittel eine gute Phasenstabilität.

Aufgrund der gefundenen rezeptiven Lösung können höherviskosere Zusammensetzungen bei Beibehaltung guter Schaumeigenschaften bzw. guter Schaumverarbeitung dadurch zum 1-Komponenten-Schaum verarbeitet werden, dass das nicht oder in geringeren Maße in der Polyolkomponente erforderliche verdünnend wirkende Trichlorpropylphosphat oder eine ähnlich wirkende Verbindung aus dem polyolischen Gemisch entnommen und der Isocyanatzubereitung zugesetzt werden kann.

Bei der Herstellung der 1-Komponenten-Schäume erfolgt die Umsetzung der Isocyanatkomponente (B) mit der Polyolkomponente (A) in Anwesenheit eines Treibmittels in einem Druckbehälter, vorzugsweise in einer Aerosoldose. Hierzu werden die Komponenten in dem oben angegebenen Verhältnis zusammen mit einem Treibmittel in einen Druckbehälter gefüllt, so dass im Druckbehälter das erfindungsgemäße Prepolymer mit einem geringeren Gehalt an freien Isocyanatgruppen entsteht. Übliche Treibmittel zur Herstellung der 1-Komponenten-Montageschäume sind beispielsweise R134a (Tetrafluorethan); R152a (1,1-Difluorethan); Dimethylether; Propan; n-Butan; iso-Butan (vorzugsweise Gemische aus Propan; n-Butan; iso-Butan).

Zur Herstellung der 1-Komponenten-Schäume wird der Druckbehälter entspannt. Dabei wird das austretende Prepolymer durch die Froth-Wirkung des Treibmittels aufgeschäumt und härtet durch die Luftfeuchtigkeit aus.

Bei der Herstellung der erfindungsgemäßen Hartschaumstoffe wird, wie allgemein bekannt, die Isocyanatkomponente (B) mit den Verbindungen mit mindestens zwei mit Isocyanatgruppen reaktiven Wasserstoffatomen im Beisein von Katalysatoren und Treibmitteln zur Umsetzung gebracht. Die Verbindungen mit mindestens zwei mit Isocyanatgruppen reaktiven Wasserstoffatomen und die Katalysatoren wurden oben beschrieben.

Als Treibmittel kann Wasser verwendet werden, das mit Isocyanatgruppen unter Abspaltung von Kohlendioxid reagiert. In Kombination mit oder an Stelle von Wasser können auch sogenannte physikalische Treibmittel eingesetzt werden. Dabei handelt es sich um gegenüber den Einsatzkomponenten inerte Verbindungen, die zumeist bei Raumtemperatur flüssig sind und bei den Bedingungen der Urethanreaktion verdampfen. Vorzugsweise liegt der Siedepunkt dieser Verbindungen unter 50 °C. Zu den physikalischen Treibmitteln zählen auch Verbindungen, die bei Raumtemperatur gasförmig sind und unter Druck in die Einsatzkomponenten eingebracht bzw. in ihnen gelöst werden, beispielsweise Kohlendioxid, niedrigsiedende Alkane und Fluoralkane.

Die Verbindungen werden zumeist ausgewählt aus der Gruppe, enthaltend Alkane und/oder Cycloalkane mit mindestens 4 Kohlenstoffatomen, Dialkylether, Ester, Ketone, Acetale, Fluoralkane mit 1 bis 8 Kohlenstoffatomen, und Tetraalkylsilane mit 1 bis 3 Kohlenstoffatomen in der Alkylkette, insbesondere Tetramethylsilan.

Als Beispiele seien genannt Propan, n-Butan, iso- und Cyclobutan , n-, iso- und Cyclopentan, Cyclohexan, Dimethylether, Methylethylether, Methylbutylether, Ameisensäuremethylester, Aceton, sowie Fluoralkane, die in der Troposphäre abgebaut werden können und deshalb für die Ozonschicht unschädlich sind, wie Trifluormethan, Difluormethan, 1,1,1,3,3-Pentafluorbutan, 1,1,1,3,3-Pentafluorpropan, 1,1,1,2-Tetrafluorethan, Difluorethan und Heptafluorpropan. Besonders bevorzugt eingesetzt werden Cyclopentan und/oder n-Pentan. Die genannten physikalischen Treibmittel können allein oder in beliebigen Kombinationen untereinander eingesetzt werden.

Die Viskosität der Iscyanatkomponente (B) ist höher als bei den üblicherweise für die Herstellung dieser Produkte eingesetzten Polyisocyanaten. Um diesen Nachteil auszugleichen, ist es möglich, die oben beschriebenen additiven Flammschutzmittel der Isocyanatkomponente (B) zuzusetzen und so deren Viskosität abzusenken.

Zur Herstellung der Polyurethan-Hartschaumstoffe werden die Polyisocyanate (B) die Polyolkomponente (A) in solchen Mengen zur Umsetzung gebracht, daß der Isocyanatindex in einem Bereich zwischen 125 und 220, vorzugsweise zwischen 145 und 195, liegt.

Die Polyurethan-Hartschaumstoffe können diskontinuierlich oder kontinuierlich mit Hilfe bekannter Mischvorrichtungen hergestellt werden.

Üblicherweise werden die erfindungsgemäßen PUR-Hartschaumstoffe nach dem Zweikomponenten-Verfahren hergestellt. Bei diesem Verfahren werden die Verbindungen mit mindestens zwei gegenüber Isocyanatgruppen reaktiven Wasserstoffatomen (A), mit den Flammschutzmitteln, den Treibmitteln, den Katalysatoren sowie den weiteren Hilfs- und/oder Zusatzstoffen zu der sogeannnten Polyolkomponente vermischt und diese mit den Polyisocyanaten oder Mischungen aus den Polyisocyanaten und gegebenenfalls Flammschutzmitteln und Treibmitteln, auch als Isocyanatkomponente bezeichnet, zur Umsetzung gebracht.

Die Ausgangskomponenten werden zumeist bei einer Temperatur von 15 bis 35 °C , vorzugsweise von 20 bis 30 °C gemischt. Das Reaktionsgemisch kann mit Hoch- oder Niederdruckdosiermaschinen in geschlossene Stützwerkzeuge gegossen werden. Nach dieser Technologie werden z. B. diskontinuierlich Sandwichelemente gefertigt.

Überraschenderweise weisen die nach dem erfindungsgemäßen Verfahren hergestellten Schaumstoffe eine sehr helle, teilweise sogar weiße Farbe auf. Die Schaumstoffe sind dimensionsstabil und auf lassen sich sehr gut applizieren.

Die Erfindung soll mit dem nachfolgenden Ausführungsbeispiel näher erläutert werden.

Ausführungsbeispiel 1 (1-Komponenten-PUR-Aerosolschaum)

### 1.1 Herstellung der Polyolkomponente:

### 1.1.1 Polyolkomponente1 (mit erhöhtem Anteil an verdünnenden Zusatz)

Aus 300 g eines Polyesterpolyols auf Basis Phthalsäureanhydrid/Diethylenglykol/Polyethylenglykol mit einem Molekulargewicht von 470g/mol, 208 g eines Polyetherpolyols auf Basis Glyzerin/Propylenoxyd/Ethylenoxyd mit einem Molekulargewicht von 4000g/mol, 30g eines Polyetherpolyols auf Basis Saccharose/Pentandiol/Diethylenglykol mit einem mittleren Molekulargewicht von 540g/mol 40 g eines Polyethylenglykols mit einem Molekulargewicht von 600g/mol, 59 g eines monofunktionellen methylierten Polyethylenglykols mit einem Molekulargewicht von 500g/mol, 25 g eines Schaumstabilisators, 330 g Trichlorpropylphoshat, 8 g Dimorpholinodiethylether, 0,5 g Siliconöl wurde durch vermischen eine Polyolkomponente hergestellt.

Sie enthält 63,6 Gew.-% A1 und A2, wobei die Bestandteile (A1) und (A2) im Verhältnis 1 : 1,1 darin enthalten sind.

### 1.1.2 Polyolkomponente2 (mit reduziertem Anteil an verdünnenden Zusatz)

Aus 164 g eines Polyesterpolyols auf Basis Phthalsäureanhydrid/Diethylenglykol/Polyethylenglykol mit einem Molekulargewicht von 470g/mol, 390 g eines Polyetherpolyols auf Basis Glyzerin/Propylenoxyd/Ethylenoxyd mit einem Molekulargewicht von 4000g/mol, 20 g eines bromierten Polyetherpolyols mit einem mittleren Molekulargewicht von 500g/mol, 80 g eines Polyethylenglykols mit einem Molekulargewicht von 600g/mol, 160g eines monofunktionellen methylierten Polyethylenglykols mit einem Molekulargewicht von 500g/mol, 25 g eines Schaumstabilisators, 150 g Trichlorpropylphoshat, 10 g Dimorpholinodiethylether, 0,2 g Paraffinöl und 0,8 g Siliconöl wurde durch Vermischen eine Polyolkomponente hergestellt.

Die Polyolkomponente (A) enthält das 81,4 Gew.-% A1 und A2, wobei die Bestandteile (A1) und (A2) im Verhältnis 1 : 4 darin enthalten sind.

### 1.2 Herstellung der Isocyanatzubereitungen:

### 1.2.1 Isocyanatzubereitung 1 (ohne verdünnenden Zusatz)

Aus Polyphenylenpolymethylenpolyisocyanat (Handelsname: Lupranat® M20) mit einem Monomer-MDI-Gehalt von 37 %, einem NCO-Gehalt von 31,2 Gew.-%, einer Viskosität von 213 mPa.s bei 25 °C wurde in einem einstufigen Extraktionsprozess, wie im nachfolgenden beschrieben, mit Cyclohexan extrahiert.

Polyphenylenpolymethylenpolyisocyanat wurde mit Cyclohexan im Verhältnis von Isocyanat : Lösungsmittel von 1 : 3 bei 50 °C für 60 min in Kontakt gebracht. Danach wurde das Produktgemisch bis zur vollständigen Phasenbildung bei Raumtemperatur stehengelassen.

Die obere Phase war das sogenannte "Extrakt", dass das gewünschte Polyphenylenpolymethylenpolyisocyanat und Lösungsmittel enthielt. Das Lösungsmittel wurde mittels Vakuumdestillation vollständig entfernt (Restgehalt an Cyclohexan kleiner 20 ppm).

Nach anschließender Entmonomerisierung in einer einstufigen Kurzwegverdampferapparatur wurde ein monomerarmes noch 424 ppm MDI enthaltendes Gemisch mit den gewünschten höherkondensierten 3- bis 5-Kern Homologen des Diphenylmethandiisocyanats mit einem NCO-Gehalt von 32,2 Gew.-% und einer Viskosität von 1260 mPa.s bei 25 °C erhalten.

Die Bestandteile an höheren Homologen des Diphenylmethandiisocyanats waren in diesem Gemisch in den Anteilen
(B1) das 3-Kern-Produkt des Polyphenylenpolymethylenpolyisocyanat 64,1 Gew. %
(B2) das 4-Kern-Produkt des Polyphenylenpolymethylenpolyisocyanat 22,7 Gew.%
(B3) das 5-Kern-Produkt des Polyphenylenpolymethylenpolyisocyanat 6,5 Gew.%
enthalten, woraus ein Verhältnis der Bestandteile (B1) : (B2) : (B3) = 9,9 : 3,5 : 1 resultierte.

Das nach obigen Angaben gefertigte Produkt wurde in dieser Form als Isocyanatzubereitung1 für die nachfolgende 1-Komponenten-Montageschaum-Fertigung verwendet.

### 1.2.2 Isocyanatzubereitung 2 (mit verdünnenden Zusatz)

Hierfür wurden 880 g der Isocyanatzubereitung1 (beschrieben im Ausführungsbeispiel 1.2.1) mit 120 g Trichlorpropylphosphat vermischt. Daraus resultierte eine Isocyanatzubereitung2 mit einer Viskosität von 890 mPas (bei 25 °C) und einem NCO-Gehalt von 28,1 Gew.%

### 1.3 Herstellung des hellen dimensionsstabilen 1-Komponenten-Montageschaumes

### 1.3.1 1-Komponenten-Montageschaum aus Polyolkomponente 1 und Isocyanatzubereitung 1

247 g der Polyolkomponente1 (nach Ausführungsbeispiel 1.1.1) wurden in eine 1-Liter-Aerosoldose gefüllt. Nach Zugabe von 383 g der Isocyanatzubereitung1 (nach Ausführungsbeispiel 1.2.1) wurde die Aerosoldose mit einem Kippventil, mittels einer zur Laborfertigung von Aerosoldosen geeigneten Apparatur, verschlossen.

Durch das Ventil wurden nachfolgend 56 g Dimethylether, 38 g einer Propan/Butan-Mischung (20 % Propan; 80 % Butan) sowie 94 g Tetrafluorethan (R134a) dosiert und der Inhalt durch schütteln homogenisiert.

Durch Wärmelagerung bei ca. 50 °C der so gefertigten Aerosoldose wurde eine künstliche Alterung erzielt, so dass die gefertigte Aerosoldose schon nach 24 h Lagerung und Abkühlung auf Raumtemperatur getestet werden konnte.

Zu diesem Zweck wurde auf einem ebenen Untergrund aufgetragenes Papiervlies angefeuchtet und der Inhalt der Aerosoldose mittels Betätigung des Kippventils mit aufgeschraubten Schaumröhrchen als schäumendes Gemisch ausgetragen.

Der Austrag erfolgt in Schaumsträngen, wobei zwischen den in Lagenform ausgetragenen Schaumsträngen eine Benetzung der Schaumoberfläche mit Wasser erfolgte.

Der ausgehärtete Schaum wurde auf seine Eigenschaften geprüft. (siehe Tabelle 1.4)

### 1.3.2 1-Komponenten-Montageschaum aus Polyolkomponente 2 und Isocyanatzubereitung 2

208 g der Polyolkomponente 2 (nach Ausführungsbeispiel 1.1.2) wurden in eine 1-Liter-Aerosoldose gefüllt. Nach Zugabe von 416 g der Isocyanatzubereitung 2 (nach Ausführungsbeispiel 1.2.2) wurde die Aerosoldose mit einem Kippventil, mittels einer zur Laborfertigung von Aerosoldosen geeigneten Apparatur, verschlossen.

Durch das Ventil wurden nachfolgend 56 g Dimethylether, 37 g einer Propan/Butan-Mischung (20 % Propan; 80 % Butan) sowie 93 g Tetrafluorethan (R134a) dosiert und der Inhalt durch schütteln homogenisiert.

Durch Wärmelagerung bei ca. 50 °C der so gefertigten Aerosoldose wurde eine künstliche Alterung erzielt, so dass die gefertigte Aerosoldose schon nach 24 h Lagerung und Abkühlung auf Raumtemperatur getestet werden konnte.

Zu diesem Zweck wurde auf einem ebenen Untergrund aufgetragenes Papiervlies angefeuchtet und der Inhalt der Aerosoldose mittels Betätigung des Kippventils mit aufgeschraubten Schaumröhrchen als schäumendes Gemisch ausgetragen.

Der Austrag erfolgt in Schaumsträngen, wobei zwischen den in Lagenform ausgetragenen Schaumsträngen eine Benetzung der Schaumoberfläche mit Wasser erfolgte.

Der ausgehärtete Schaum wurde auf seine Eigenschaften geprüft. (siehe Tabelle 1.4)

### 1.4 Eigenschaften der 1-Komponenten-Montageschäume

| | Schaum nach 1.3.1 | Schaum nach 1.3.2 | Vergleich mit handelsüblichen Schaum*** |
|---|---|---|---|
| Verarbeitung* | erhöhte Frothwirkung | erhöhte Frothwirkung | geringere Frothwirkung |
| Farbe | weiß | weiß | gelb/braun |
| Zugfestigkeit | 15,6 N/cm² | 10,5 N/cm² | 8 N/cm² |
| Bruchdehnung | 17 % | 18 % | 20 % |
| Druckspannung ( 10 % Verformung) | 6,9 N/cm² | 6,5 N/cm² | 5 N/cm² |
| Dimensionsstabilität** | kein Schrumpf | kein Schrumpf | - 4,2% |

| | | | |
|---|---|---|---|
| Verarbeitung* = Frothwirkung beschreibt das Expansionsverhalten des mit Flüssiggas als Treibmittel beladenen Prepolymers beim unmittelbaren Austrag aus der Aerosoldose Dimensionsstabilität** gemessen an Prüfkörpern aus zwei Spanplatten plus Distanzstäben und dazwischen eingetragenen und ausgehärteten Schaum. Nach der Aushärtung des Schaumes und Entfernung der Distanzstäbe wurde die %-tuale Änderung des Plattenabstandes als Maß der Dimensionsstabilität gemessen. *** Schaum mit Lupranat® M20 als Isocyanatkomponente Ausführungsbeispiel 2 (2-Komponenten-PUR-Hartschaum) | | | |

### 2.1 Herstellung der Polyolkomponente

Aus 377 g Lupranol^{®} 3424 (Polyetherpolyol auf Basis Saccharose, Pentaerythrit, Diethylenglyol und Propylenoxyd mit einer OH-Zahl von 403 mgKOH/g), 230 g Lupranol^{®} 3423 (Polyetherpolyol auf Basis Saccharose, Glycerin und Propylenoxyd mit einer OH-Zahl von 490 mgKOH/g), 20 g Glyzerin, 300 g Lupranol^{®} 1100 (Polyetherpolyol auf Basis Propylenglykol und Propylenoxyd mit einer OH-Zahl von 104 mgKOH/g), 54 g Lupranol^{®} VP9319 (Polyetherpolyol auf Basis Trimethylolpropan und Propylenoxyd mit einer OH-Zahl von 160 mgKOH/g), 10 g Stabilisator Tegostab^{®} B8443, 5 g Stabilisator Niax Silicone SR 393 und 4,5 g Wasser wurde ein Polyolgemisch hergestellt. Diesem Gemisch wurden sowohl 34 g eines Katalysatorgemisches (23,3 % N,N-Dimethylcyclohexylamin, 18,7 % 1-Methylimidazol, 28 % Tetramethylhexandiamin und 30 % Lupranol^{®} 1200 [Polyetherpolyol auf Basis Propylenglykol und Propylenoxyd mit einer OH-Zahl von 248 mgKOH/g]) als auch 50 g eines wässrigen Glyzerin/Glykol-Gemisches (enthaltend 9 % Gyzerin und 31 % Dipropylenglykol) zugesetzt und daraus die Polyolkomponente gefertigt.

### 2.2 Isocyanatkomponente

Dazu wurde die Isocyanatkomponente gemäß Ausführungsbeispiel 1.2.1 zu 85 Gew.% mit 15 Gew.% Triethylphosphat gemischt. Man erhielt eine Isocyanatkomponente mit folgenden Eigenschaften:

| | |
|---|---|
| Viskosität (25 °C) | 477 mPas |
| Isocyanatgehalt | 27,2 % NCO |

### 2.3 Verarbeitung der Komponenten zu PUR-Hartschaum

Die Komponenten nach Ausführungsbeispiel 2.1 bzw. 2.2 wurden im Mischungsverhältnis Polyol- : Isocyanatkomponente = 100 : 164 gemischt und nach Aufschäumen und Aushärtung wurde ein weißer Hartschaum erhalten. Der Schaum hatte dabei (freigeschäumt) folgende Eigenschaften:

| | |
|---|---|
| Startzeit: | 15 sec |
| Abbindezeit: | 46 sec |
| Steigzeit: | 84 sec |
| Raumgewicht: | 43 kg/m³ |
| Druckfestigkeit: | 31,8 N/cm² |

| | Schaum nach Ausführungsbeispiel 2.3 | Schaum unter Verwendung von Lupranat^{®} M20 (analoge Polyolkomponente) |
|---|---|---|
| Dimensionsstabilität (Breite) | ± 0 (kein Schrumpf) | - 0,2 %* |
| Dimensionsstabilität (Länge) | ± 0 (kein Schrumpf) | - 0,6 %* |

## Patentansprüche

1. Isocyanatkomponente (B), enthaltend höhere Homologe des Diphenylmethandiisocyanats, enthaltend
(B1) das 3-Kern-Produkt des Polyphenylenpolymethylenpolyisocyanats,
(B2) das 4-Kern-Produkt des Polyphenylenpolymethylenpolyisocyanats,
(B3) das 5-Kern-Produkt des Polyphenylenpolymethylenpolyisocyanats
wobei diese in einem Gewichtsverhältnis der Komponenten (B1) : (B2) : (83) = 8,0±4,0 : 3,5±1,8 : 1,2±0,9 vorliegen, und die Isocyanatkomponente (B) einen Gehalt an Di-phenylmethandiisocyanat von maximal 2 Gew.-% und einen Gehalt an Uretoniminen von maximal 6 Gew.-%, jeweils bezogen auf das Gewicht der Isocyanatkomponente (B), aufweist.

2. Isocyanatgruppen und Urethangruppen enthaltende Prepolymere, hergestellt durch Umsetzung einer Polyolkomponente (A) und einem stöchiometrischen Überschuss einer Isocyanatkomponente (B), **dadurch gekennzeichnet, dass**
die Isocyanatkomponente (B) höhere Homologe des Diphenylmethandiisocyanats, enthaltend
(B1) das 3-Kern-Produkt des Polyphenylenpolymethylenpolyisocyanats,
(B2) das 4-Kern-Produkt des Polyphenylenpolymethylenpolyisocyanats,
(B3) das 5-Kern-Produkt des Polyphenylenpolymethylenpolyisocyanats
wobei diese in einem Gewichtsverhältnis der Komponenten (B1) : (B2) : (B3) = 8,0±4,0 : 3,5±1,8 : 1,2±0,9 vorliegen, und die Isocyanatkomponente (B) einen Gehalt an Diphenylmethandiisocyanat von maximal 2 Gew.-% und einen Gehalt an Uretoniminen von maximal 6 Gew.-%, jeweils bezogen auf das Gewicht der Isocyanatkomponente (B), aufweist,
enthält.

3. Isocyanatgruppen und Urethangruppen enthaltende Prepolymere nach Anspruch 2, **dadurch gekennzeichnet, dass** die Polyolkomponente (A) ein Gemisch aus
(A1) einem Polyesterpolyol mit einem Molekulargewicht von maximal 600 g/mol und
(A2) einem Polyetherpolyol oder Polyetherpolyolgemisch mit einem mittleren Molekulargewicht von 1000 bis 5000 g/mol,
enthält.

4. Isocyanatgruppen und Urethangruppen enthaltende Prepolymere nach Anspruch 2 und 3, **dadurch gekennzeichnet, dass** als Polyesterpolyol (A1) ein Polyesterpolyol auf Basis Phthalsäureanhydrid/Diethylenglykol/Polyethylenglykol verwendet wird.

5. Isocyanatgruppen und Urethangruppen enthaltende Prepolymere nach Anspruch 2 und 3, **dadurch gekennzeichnet, dass** die Bestandteile (A1) und (A2) im Verhältnis 1 : 6 bis 3 : 1 in der Polyolkomponente (A) eingesetzt werden.

6. Isocyanatkomponente (B) nach Anspruch 1, **dadurch gekennzeichnet, dass** die Isocyanatkomponente (B) zu mehr als 85 Gew.-%, bezogen auf das Gewicht der Isocyanatkomponente (B), aus den Komponenten (B1), (B2) und (B3) besteht.

7. Isocyanatkomponente (B) nach Anspruch 1, **dadurch gekennzeichnet, dass** die Isocyanatkomponente (B) eine Farbzahl von kleiner 5 Jod, einen L*-Wert von größer 96 und einen b*-Wert von kleiner 15 aufweist, bestimmt nach DIN 6162 und DIN 6164.

8. Verwendung der Isocyanatkomponente (B) oder der Isocyanatgruppen und Urethangruppen enthaltenden Prepolymere nach den Ansprüchen 1 bis 7 zur Herstellung von hellen Polyurethanschaumstoffen.

9. Verwendung der Isocyanatgruppen und Urethangruppen enthaltendes Prepolymer nach den Ansprüchen 1 bis 7 zur Herstellung von 1-Komponenten-Montageschaumstoffen.

10. Verwendung der Isocyanatkomponente (B) gemäß Anspruch 1 zur Herstellung von Polyurethan-Hartschaumstoffen.

11. Verfahren zur Herstellung von 1-Komponenten-Montageschaumstoffen durch Umsetzung einer Polyolkomponente (A) und einem stöchiometrischen Überschuss einer Isocyanatkomponente (B), **dadurch gekennzeichnet, dass** die Isocyanatkomponente (B) höhere Homologe des Diphenylmethandiisocyanats, enthaltend
(B1) das 3-Kern-Produkt des Polyphenylenpolymethylenpolyisocyanats,
(B2) das 4-Kern-Produkt des Polyphenylenpolymethylenpolyisocyanats,
(B3) das 5-Kern-Produkt des Polyphenylenpolymethylenpolyisocyanats
wobei diese in einem Gewichtsverhältnis der Komponenten (B1) : (B2) : (B3) = 8,0±4,0 : 3,5±1,8 : 1,2±0,9 vorliegen, und die Isocyanatkomponente (B) einen Gehalt an Diphenylmethandiisocyanat von maximal 2 Gew.-% und einen Gehalt an Uretoniminen von maximal 6 Gew.-%, jeweils bezogen auf das Gewicht der Isocyanatkomponente (B), aufweist und die Komponenten (A) und (B) in Gegenwart von Flüssiggasen in einem Druckbehälter umgesetzt werden.

12. Verfahren nach Anspruch 11, **dadurch gekennzeichnet, dass** die Polyolkomponente (A) ein Gemisch aus
(A1) einem Polyesterpolyol mit einem Molekulargewicht von maximal 600 g/mol und
(A2) einem Polyetherpolyol oder Polyetherpolyolgemisch mit einem mittleren Molekulargewicht von 1000 bis 5000 g/mol
enthält.

13. Verfahren zur Herstellung von Polyurethan-Hartschaumstoffen durch Umsetzung von Verbindungen mit mindestens zwei mit Isocyanatgruppen reaktiven Wasserstoffatomen mit einer Isocyanatkomponente (B), **dadurch gekennzeichnet, dass** die Isocyanatkomponente (B) höhere Homologe des Diphenylmethandiisocyanats enthält, enthaltend
(B1) das 3-Kern-Produkt des Polyphenylenpolymethylenpolyisocyanats,
(B2) das 4-Kern-Produkt des Polyphenylenpolymethylenpolyisocyanats,
(B3) das 5-Kern-Produkt des Polyphenylenpolymethylenpolyisocyanats
wobei diese in einem Gewichtsverhältnis der Komponenten (B1) : (B2) : (B3) = 8,0±4,0 : 3,5±1,8 : 1,2±0,9 vorliegen, und die Isocyanatkomponente (B) einen Gehalt an Diphenylmethandiisocyanat von maximal 2 Gew.-% und einen Gehalt an Uretoniminen von maximal 6 Gew.-%, jeweils bezogen auf das Gewicht der Isocyanatkomponente (B), aufweist.
